# FASCICULE DE BREVET EUROPEEN

(11) **EP 0 734 720 B1**
(45) Date de publication et mention de la délivrance du brevet: **12.07.2000**
(21) Numéro de dépôt: 96420098.4
(22) Date de dépôt: 28.03.1996
(51) Int. Cl.: A61K 9/16, A61K 9/51

(54) **Particules à base de polyaminoacide(s) et susceptibles d'être utilisées comme vecteurs de principe(s) actif(s) et leurs procédés de préparation**
Partikel auf Polyaminosäure(n)basis zur Verwendung als Wirkstoffträger sowie deren Herstellungsverfahren
Particles based on polyamino-acid(s) for use as carriers for active agents and their processes of preparation

(30) Priorité: 28.03.1995 FR 9503978
(43) Date de publication de la demande: 02.10.1996
(73) Titulaire: FLAMEL TECHNOLOGIES, 69693 Venissieux Cédex (FR)
(72) Inventeur: Huille, Sylvain, 69008 Lyon (FR); Lemercier, Alain, 69720 St Bonnet de Mure (FR); Soula, Gérard, 69330 Meyzieu (FR)
(74) Mandataire: Fleurance, Raphael

(56) Documents cités:
- US-A- 4 351 337
- US-A- 4 976 968
- PHARM. ACTA HELV. , vol. 58, no. 7, 1983, CH, pages 196-209, XP002007426 J. KREUTER : "EVALUATION OF NANOPARTICLES AS DRUG-DELIVERY SYSTEMS I: PREPARATION METHODS"

## Description

Le domaine de la présente invention est celui des vecteurs utiles pour l'administration de principes actifs **(PA)** à travers des membranes cellulaires. Ces vecteurs permettent le transport sous protection des **PA**, à l'intérieur d'un organisme, jusqu'à leur site d'action. Le **PA** est, de préférence, un médicament ou un nutriment pour l'administration à un organisme animal ou humain par voir orale, nasale, vaginale, oculaire, sous-cutanée, intraveineuse, intramusculaire, intradermique, intrapéritonéale, intracérébrale, parentérale, etc., mais il peut être aussi un herbicide, un pesticide, un insecticide, un fongicide, etc., pour le traitement des cultures agricoles comme application phytosanitaire. Pour toutes ces applications, les vecteurs de **PA** visent à améliorer la biodisponibilité des **PA**. Ces vecteurs peuvent être, e. g., des systèmes à libération prolongée de **PA**.

Les **PA** plus particulièrement, mais non limitativement, concernés par l'invention sont, par exemple, des protéines, des glycoprotéines, des peptides, des polysaccharides, des lipopolysaccharides, des oligonucléotides et des polynucléotides.

La présente invention concerne, plus précisément, des particules - avantageusement de type submicroniques et/ou microniques - à base de polyaminoacides et destinées à être utilisées comme vecteurs de **PA**, en particulier médicamenteux. Ce sont donc des Particules de Vectorisation **(PV)**, parmi lesquelles on distinguera, dans la suite du présent exposé, d'une part, des Nano Particules de Vectorisation **(NPV)** et, d'autre part, des MicroParticules de Vectorisation **(MPV)**, selon une nomenclature propre à l'invention et qui sera définie infra.

La présente invention vise aussi bien les particules nues en tant que telles, que les systèmes de vecteurs de **PA**, constitués par les particules chargées par le (ou les) **PA** considéré(s).

L'invention concerne, également, un procédé de préparation desdites particules.

Les progrès du génie génétique et des biotechnologies, ainsi que les découvertes y afférentes d'outils génétiques, de protéines et de peptides biologiquement actifs, ont permis l'essor de nouveaux principes actifs médicamenteux **(PA)** offrant une activité intrinsèque et une sélectivité élevées. Ces **PA** sont, en revanche, facilement dégradés dans l'organisme avant d'atteindre leur site d'action thérapeutique et leur biodisponibilité est, en conséquence, très faible. Dans le cas de l'administration par la voie orale, le tractus gastro-intestinal constitue une barrière chimique et physique redoutable pour le **PA** qui doit, d'une part, résister à la dégradation par le système digestif et, d'autre part, passer à travers la membrane épithéliale gastro-intestinale. A cet égard, on pourra, par exemple, se reporter à la revue de M. J. HUMPHREY (Delivery System for peptide Drugs, éditée par S. DAVIS et L. ILLUM, Plenum Press, N. Y., 1986), qui fait état de la faible biodisponibilité des peptides et, en particulier, des peptides administrés par voie orale.

Naturellement, ces avatars de transport et de séjour dans l'organisme ne se limitent pas aux protéines, mais affectent également les **PA** formés par des outils génétiques (oligonucléotides, polynucléotides, plasmides) susceptibles d'être mis en oeuvre dans les techniques de thérapie génique.

Pour pallier cela, il a été proposé d'encapsuler les **PA** dans des particules de vectorisation de **PA**, dénommées également **PV**. L'intérêt de ces techniques d'encapsulation est de protéger et/ou de transporter le **PA** jusqu'à son site d'action thérapeutique, en le sauvegardant contre les agressions de l'organisme, afin d'augmenter sa biodisponibilité.

Parmi tous les matériaux envisageables pour l'encapsulation de **PA**, les polymères sont de plus en plus utilisés, du fait de leurs propriétés intrinsèques.

S'agissant du cahier des charges que l'on souhaite obtenir pour de telles **PV**, il est particulièrement exigeant et comprend, notamment, les spécifications suivantes.
**1** - Il devrait, avantageusement, être possible de pouvoir disposer de **PV** de diamètre moyen compris entre une fraction de micron et quelques microns, avec une répartition granulométrique étroite, de façon à pouvoir adapter la granulométrie des **PV** au mode d'administration choisi et/ou le site thérapeutique visé. Par exemple, si une immunisation mucosale par la voie orale est recherchée, la taille des **PV** doit être comprise entre 0,5 µm et 10 µm, afin que les **PV** puissent pénétrer les plaques de Peyer et atteindre les tissus lymphoïdes. Dans le cas d'une administration sous-cutanée, il y a avantage à disposer de **PV** de taille supérieure à 10 µm pour que les particules ne rentrent pas dans la circulation générale où elles sont rapidement internalisées par le système réticulo-endothélial, mais qu'elles diffusent progressivement depuis leur site d'injection.
   Cette spécification implique un contrôle dimensionnel des **PV**, à la fois sur la distribution de la granulométrie des **PV** et sur leur diamètre moyen, qui représente une opération très délicate sur le plan technologique.
**2 -** Il est souhaitable que les **PV** assurent la protection du **PA** jusqu'au site de libération. Par exemple, dans une administration orale d'un **PA** formé par un vaccin, ce dernier gagnerait à être protégé, tout au long du tractus gastrointestinal.
**3 -** Il est préférable que le polymère, constituant les **PV**, soit biocompatible et biodégradable et, encore mieux, qu'il soit métabolisé en produits non toxiques pour l'organisme.
**4 -** Il est également avantageux que le polymère, constitutif des **PV**, n'induise pas de réponse immunitaire (immunogéniale).
**5 -** Enfin, il est également préférable que les **PV** puissent être obtenues par un procédé non dénaturant pour le **PA**. Ainsi, l'usage de solvants organiques et/ou de températures élevées est à proscrire.

De nombreuses propositions techniques antérieures ont vainement tenté de satisfaire à l'ensemble de ces spécifications. Les réponses apportées jusqu'alors ne sont donc que partielles et incomplètes.

Parmi ces propositions infructueuses, on peut citer celle selon le brevet US-A-5 286 495, qui concerne un procédé d'encapsulation de protéines en phase aqueuse, à l'aide de matériaux constitués d'alginate et de polylysine. Ce procédé est présenté comme non dénaturant pour les **PA** protéiniques, en raison du fait qu'il n'y est pas fait usage de solvant organique, de réactif chimique agressif ou de température élevée. Cependant, la technique de fabrication des **PV**, par vaporisation mise en oeuvre, ne permet pas de produire des particules de taille inférieure à 35 µm, ce qui ne permet pas leur internalisation par les cellules de l'organisme.

Par ailleurs, les techniques d'émulsion sont couramment utilisées pour préparer des microparticules de quelques microns.

Par exemple, les demandes de brevets WO 91/06 286 et WO 91/06 287 décrivent des procédés de formation de particules en émulsion dans lesquels on utilise, comme polymère :
- soit une protéine hydrophobe choisie parmi le collagène, la caséine, la kératine et, de préférence, les prolamines,
- soit un polymère biocompatible et biodégradable, tel que les poly(lactiques) ou les poly(orthoesters).

Le **PA** peut être hydrophobe ou hydrophile mais, dans ce dernier cas, la technique de double émulsion est recommandée. La taille des microparticules est d'environ 100 µm et, de préférence, comprise entre 50 nm et 100 µm.

La demande de brevet WO 89/08 449 fait également référence à l'encapsulation par émulsion, pour incorporer des **PA** dans des microparticules de poly(lactiques) de taille inférieure à 10 µm. Et il est précisé, dans ce document, que cette taille est une limite maximale pour l'absorption au travers des tissus lymphoïdes des muqueuses (administrations orale, nasale, rectale, ophtalmologique).

Les techniques d'émulsion sont très séduisantes a priori, car elles permettent la mise en oeuvre de la plupart des **PA** dans des microparticules, dont on peut contrôler la granulométrie jusqu'à des tailles de l'ordre de 1 µm. Mais, dans ces techniques, on a recours à des solvants organiques pour solubiliser les polymères constitutifs des particules. Ces solvants sont, e. g. des cétones, des alcools, des amides ou leurs mélanges. Et malheureusement, il s'avère que ces solvants peuvent être dénaturants, notamment pour les **PA** peptidiques ou polypeptidiques.

On connaît, également, des **PV** biocompatibles, formées en solution aqueuse sans élévation excessive de la température et appelées protéinoïdes. Ces **PV** ont été décrites dès 1970 par W. FOX et K. DOSE dans "Molecular Evolution and the origin of Life", Ed. Marcel DEKKER Inc. (1977).

En s'inspirant de ces travaux, la demande de brevet WO 88/01 213 ('1213) propose un système à délivrance de **PA** à base de protéinoïdes. Le polymère utilisé est un mélange de polypeptides artificiels obtenus par condensation thermique d'acides aminés synthétiques ou naturels et/ou de petites chaînes peptidiques. Le mode de condensation choisi conduit à des oligomères ramifiés et donc très peu solubles. Il est ensuite procédé à une sélection par filtration de ces oligomères ramifiés, afin de récupérer les fractions hydrosolubles. Cette fraction est nécessairement composée de réticulats ramifiés de très petite masse. Les microparticules selon cette invention sont obtenues par changement de pH qui crée la précipitation des oligomères ramifiés en protéinoïdes.

Lorsque la solution dans laquelle s'effectue la précipitation contient des **PA** en solution, une partie d'entre eux est entraînée dans le protéinoïde lors de sa formation.

Les inconvénients de ce système sont :
- un faible taux d'encapsulation,
- un procédé de purification délicat,
- un enchaînement non régulier (non alpha peptidique) des aminoacides dû au mode de synthèse qui ne permet pas d'affirmer que les réactions de dégradation enzymatiques seront identiques à celles d'un alpha-polyaminoacide,
- enfin, l'utilisation d'un grand nombre d'aminoacides monomères différents, qui peut induire une réponse immunitaire.

La demande de brevet WO 93/25 589 porte sur l'amélioration du procédé de synthèse de proténoïdes par condensation thermique d'acides aminés.

Les protéinoïdes sont, cette fois encore, formés d'oligomères ramifiés de faibles masses molaires, constitués d'enchaînements irréguliers d'aminoacides. Le caractère hydrosoluble de ces oligomères ramifiés est obtenu :
- d'une part, par l'utilisation de très faibles masses (entre 250 et 2 400), ce qui correspond à des enchaînements très courts de 2 à 20 aminoacides,
- d'autre part, par le choix des aminoacides de départ.

Comme précédemment, les protéinoïdes sont formés par précipitation déclenchée par abaissement du pH des oligomères ramifiés hydrosolubles. Lorsque cette précipitation a lieu en présence de **PA** hydrosolubles, une partie de ceux-ci est entraînée dans le protéinoïde lors de sa formation. Les taux d'encapsulation restent modestes : de 20 à 40 %. Par ailleurs, l'abaissement du pH peut être dommageable à certains **PA**.

En outre, le fait de devoir réaliser l'encapsulation à un pH particulier constitue une contrainte méthodologique gênante et limite l'utilisation de ces microparticules au pH de précipitation des protéinoïdes qui ne correspond pas nécessairement aux pH biologiques. Par exemple, le pH peut varier de 2 à 7,5 dans le tractus gastro-intestinal.

On mentionnera également, pour mémoire, le brevet US 4 351 337 qui relève d'un domaine différent de celui de la vectorisation de **PA** propre à l'invention. Ce brevet divulgue des implants fixés et localisés à des endroits bien précis de l'organisme. De tels implants n'ont donc rien à voir avec des formes administrables e. g. per os ou par injection. Lesdits implants peuvent être, entre autres, des microcapsules sphériques de type matriciel ou à gangue, dont les dimensions sont de l'ordre de 400-800 µm **(fig. 8-9)** et donc bien supérieures aux dimensions de l'ordre de 0,5 µm et 10 µm requises pour que les microparticules soient internalisées par les cellules de l'organisme. Ces implants sont réalisés à partir de matériaux polymères du genre polyaminoacide (Leu/Glu notamment). Le formage de ces implants est effectué, e. g., à l'aide de solutions de polyaminoacides dans du dioxane, que l'on évapore in fine.

Dans cet état de connaissances, l'un des objectifs essentiels de la présente invention est de fournir des **PV**, en particulier submicroniques et microniques, à base de polyaminoacides et susceptibles de servir de vecteurs d'un principe actif **(PA)**, en particulier médicamenteux et/ou nutritionnel, pour l'administration dudit **PA** à un organisme humain ou animal, ces **PV** satisfaisant pleinement au cachier des charges explicité supra et répété ci-après :
**1-** Il devrait, avantageusement, être possible de pouvoir disposer de **PV** de diamètre moyen compris entre une fraction de micron et quelques microns, avec une répartition granulométrique étroite, de façon à pouvoir adapter la granulométrie des PV au mode d'administration choisi et/ou le site thérapeutique visé. Par exemple, si une immunisation mucosale par la voie orale est recherchée, la taille des **PV** doit être comprise entre 0,5 µm et 10 µm, afin que les **PV** puissent pénétrer les plaques de Peyer et atteindre les tissus lymphoïdes. Dans le cas d'une administration sous-cutanée, il y a avantage à disposer de **PV** de taille supérieure à 10 µm pour que les particules ne rentrent pas dans la circulation générale où elles sont rapidement internalisées par le système réticulo-endothélial, mais qu'elles diffusent progressivement depuis leur site d'injection.
   Cette spécification implique un contrôle dimensionnel des **PV**, à la fois sur la distribution de la granulométrie des **PV** et sur leur diamètre moyen, qui représente une opération très délicate sur le plan technologique.
**2** - Il est souhaitable que les **PV** assurent la protection du **PA** jusqu'au site de libération. Par exemple, dans une administration orale d'un **PA** formé par un vaccin, ce dernier gagnerait à être protégé, tout au long du tractus gastrointestinal.
**3** - Il est préférable que le polymère, constituant les **PV**, soit biocompatible et biodégradable et, encore mieux, qu'il soit métabolisé en produits non toxiques pour l'organisme.
**4** - Il est également avantageux que le polymère, constitutif des **PV**, n'induise pas de réponse immunitaire (immunogéniale).
**5** - Enfin, il est également préférable que les **PV** puissent être obtenues par un procédé non dénaturant pour le **PA**. Ainsi, l'usage de solvants organiques et/ou de températures élevées est à proscrire.

Un autre objectif essentiel de l'invention est de fournir des **PV** à base de polyaminoacides qui soient d'une granulométrie moyenne contrôlable et ajustable, et ce, dans des ordres de grandeur variant de 200 µm **(MPV)** jusqu'à quelques nanomètres **(NPV)**.

Un autre objectif essentiel de l'invention est de fournir des **PV** qui soient simples à préparer (pH non agressif), stables à tout pH compris entre 4 et 13 et non immunogènes.

Un autre objectif essentiel de l'invention est de fournir des **PV** à base de polyaminoacides qui soient faisables industriellement et économiques et qui soient aptes à se charger en **PA** avec des forts taux de chargement.

Un autre objectif essentiel de l'invention est de fournir un procédé de préparation de **MPV** et/ou de **NPV** à base polyaminoacides et susceptibles d'être utilisées comme vecteurs de **PA**, ledit procédé se devant d'être économique, simple à mettre en oeuvre, non dénaturant pour les **PA** et devant, en outre, permettre une maîtrise fine de la granulométrie moyenne des particules obtenues (maximum 200 µm).

Un autre objectif essentiel de l'invention est l'utilisation des susdites particules pour la préparation de médicaments (e. g. vaccins) et/ou de nutriments, en particulier pour administration *per os,* nasale, vaginale, oculaire, sous-cutanée, intraveineuse, intramusculaire, intradermique, intrapéritonéale, intracérébrale ou parentérale, de principes actifs, tels que des protéines, des glycoprotéines, des peptides, des polysaccharides, des lipopolysaccharides, des oligonucléotides et des polynucléotides.

Un autre objectif essentiel de l'invention est de fournir un médicament du type système à libération prolongée de **PA**, qui soit biocompatible et qui procure une haute biodisponibilité du **PA**.

Un autre objectif essentiel de l'invention est de fournir un système de vectorisation de vaccins qui soit non immunogène intrinsèquement et en combinaison avec un ou plusieurs antigènes.

Les objectifs relatifs aux produits, parmi d'autres, sont atteints par la présente invention qui concerne des particules de vectorisation de principe(s) actif(s), du type de celles à base de polyaminoacide(s) et de taille moyenne inférieure à 200 µm, caractérisées :
- en ce qu'elles ont une taille moyenne comprise entre 0,01 et 0,5 µm, de préférence entre 0,03 et 0,4 µm,
- en ce que ces NPV sont obtenues par mise en contact de polyaminoacides (**PAA**) avec une solution aqueuse,
- en ce que ces **PAA** sont linéaires à enchaînements α-peptidiques et comprennent au moins deux types d'aminoacides récurrents AAN et AAI :
   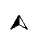 le type AAN correspondant à un acide aminé neutre hydrophobe,
   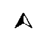 et le type AAI correspondant à un acide aminé à chaîne latérale ionisable, au moins une partie des aminoacides récurrents de type AAI étant sous forme ionisée,
      les aminoacides récurrents de chaque type AAN et AAI étant identiques ou différents entre eux,
- en ce que la masse molaire en poids M_{w} des **PAA** est supérieure ou égale à 4 000 D, de préférence à 5000 D,
- et en ce que ces **PAA** sont non-hydrosolubles à pH acide et à pH compris entre 3 et 13.

Il est du mérite de la Demanderesse d'avoir procédé à une sélection parmi les polyaminoacides, pour ne retenir que ceux ayant pour trait d'être non hydrosolubles, formant des suspensions colloïdales stables dans un large domaine de pH compatibles avec le pH des milieux physiologiques pour les applications visées et comportant un premier type de monomères AAN formé par un acide aminé neutre hydrophobe et au moins un deuxième type de monomères formé par un acide aminé AAI, caractérisé par une chaîne latérale de fonctionnalité carboxyle (Glu, Asp), ionisable à des pH physiologiques non dénaturants pour les protéines.

Selon une caractéristique de l'invention, ces polyaminoacides (**PAA**) sont linéaires et, plus préférentiellement encore, présentent des enchaînements α-peptidiques.

Avantageusement, les **PAA**, sélectionnés comme éléments constitutifs des PV de l'invention, peuvent être des **PAA** "blocs" et/ou des **PAA** "statistiques". Les **PAA** "blocs" sont ceux ayant une structure ordonnée, séquentielle et alternée, dans laquelle les aminoacides sont répartis en blocs le long des chaînes de polymères. Les **PAA** "statistiques" sont ceux ayant une structure désordonnée, séquentielle et aléatoire, dans laquelle les aminoacides sont répartis de façon non régulière le long des chaînes de polymères.

S'agissant du rapport molaire AAN / AAI + AAN, il est fonction de la structure "bloc" ou "statistique" des **PAA**. Ainsi, ce rapport molaire est :
- ≥ 6 %, de préférence ≥ 15 % pour les **PAA** "blocs",
- ≥ 20 %, de préférence ≥ 25 % pour les **PAA** "statistiques".

Selon une autre caractéristique de l'invention, la masse des polyaminoacides sélectionnés est élevée.

A cet égard, il convient de signaler que la masse molaire en poids (M_{w}) préférée, pour les polyaminoacides mis en oeuvre dans le cadre de l'invention, se définit différemment en fonction du type de polyaminoacide envisagé.
C'est ainsi que M_{w} ≥ 5 500 D, de préférence est comprise entre 6 500 D et 200 000 D et, plus préférentiellement encore, entre 8 000 et 20 000 D, pour les polyaminoacides "blocs", tels que définis supra.
Tandis que, pour les polyaminoacides "statistiques", définis eux aussi supra, M_{w} ≥ 10 000 D, de préférence comprise entre 20 000 D et 500 000 D et, plus préférentiellement, entre 20 000 D et 150 000 D.

Ces polyaminoacides forment des polymères amphiphiles pouvant interagir, à la fois avec des substances hydrophobes et des substances hydrophiles, ce qui leur confère des propriétés remarquables comme tensioactifs ou comme dispersants. Mais, en plus de leurs propriétés amphiphiles, ces polyaminoacides se distinguent par une propriété nouvelle et tout à fait inattendue, les chaînes de polyaminoacides en solution aqueuse s'associent spontanément et forment des particules qui peuvent s'associer avec des protéines. En pratique, ces particules forment, de préférence, des matrices au sein desquelles sont dispersés le (ou les) **PA**. La structure linéaire préférée par enchaînements α-peptidiques et la masse molaire élevée sont aussi des caractéristiques importantes de ces polyaminoacides.

Ces **PAA** non hydrosolubles se distinguent par une propriété nouvelle et tout à fait inattendue. Mis au contact d'une solution aqueuse, ils forment spontanément, dans celle-ci, une suspension colloïdale de nanoparticules **(NPV)** susceptibles de s'agréger en microparticules **(MPV)**. En outre, des protéines en solution peuvent s'associer spontanément avec ces particules pour former des particules chargées de **PA**.

Cette découverte est d'autant plus surprenante que l'enseignement de la demande WO 93/25 583 était plutôt de nature à inciter l'homme du métier à orienter ses recherches d'un matériau idéal pour l"'encapsulation" de protéines, vers d'autres produits que les polyaminoacides. En effet, les nombreux essais, réalisés et donnés dans la demande de brevet WO 93/25 583, laissent à penser que, parmi tous les polyaminoacides testés, seuls ceux sélectionnés et revendiqués conviennent. Ce n'est qu'au terme d'une démarche inventive que la Demanderesse a pu prouver qu'il n'en était rien en proposant une autre sélection de polyaminoacides ayant un comportement différent de ceux selon le WO 93/25 583, ces **PAA** étant, en particulier :
- des **PAA** linéaires et de masses molaires élevées (supérieures à 4 000 D), plutôt que des petits oligomères ramifiés,
- des **PAA** insolubles, plutôt que solubles, et il est surprenant que ces **PAA** insolubles forment spontanément une suspension colloïdale de **NPV** et que des protéines s'associent spontanément à ces **NPV**.

Les polyaminoacides préférés sont des polymères synthétiques linéaires, composés, avantageusement, d'α-aminoacides liés par des liaisons peptides. Il existe de nombreuses techniques synthétiques pour former des polymères à blocs ou statistiques, des polymères à chaînes multiples et des polymères contenant une séquence déterminée d'aminoacides (cf. Encyclopedia of Polymer Science and Engineering, volume 12, page 786 ; John Wiley & Sons). De nombreux dérivés d'aminoacides et de peptides ont été utilisés comme monomères pour la préparation des polyaminoacides. Cependant, les monomères servant le plus couramment sont les anhydrides des N-carboxy-α-aminoacides dont la préparation est donnée, par exemple, dans Biopolymers, 15, 1869 (1976). Les techniques de polymérisation de ces monomères sont connues de l'homme de l'art et sont détaillées dans l'ouvrage de H. R. KRICHELDORF "α-Aminoacid-N-Carboxy Anhydrides and Related Heterocycles" Springer Verlag (1987);

Les techniques de synthèses impliquent, généralement, de protéger les fonctions réactives des acides aminés à chaînes latérales ionisables, afin qu'elles n'interfèrent pas lors de l'étape de polymérisation. Il s'ensuit qu'une étape de déprotection est nécessaire pour rétablir la fonctionnalité des chaînes latérales ionisables du polymère. On peut citer, par exemple, les procédés de déprotection par saponification des esters méthyliques (STAHMAN et coll ; J. Biol. Chem., 197, 771 (1952) ; KYOWA HAKKO, FR 2 152 582) ou de débenzylation [BLOUT et coll. ; J. Amer. Chem. Soc., 80, 4631 (1858)].

Avantageusement, les **PV** ont une concentration moyenne en polyaminoacide variant de 0,01 % à 25 % poids sec, de préférence de 0,05 à 10 % poids sec.

Selon un mode préféré de réalisation des particules selon l'invention, l'AAN (ou les AAN) est(sont) choisi(s) dans la liste suivante : Leu - Ile - Val - Ala-Pro - Phe - et leurs mélanges et l'AAI (ou les AAI) est (sont) formé(s) par le Glu et/ou l'Asp.

De manière plus préférée encore, les particules de l'invention sont caractérisées en ce que leurs polyaminoacides constitutifs comportent un seul type de monomères AAI correspondant, de préférence, à Glu et un seul type de monomères correspondant, de préférence, à Leu.

Le fait de limiter le nombre de comonomères à deux seulement : un de type AAN et un de type AAI, permet de minimiser l'immunogénicité des particules. Il s'agit là d'un avantage notable de cette forme préférée de réalisation de l'invention.

La taille des particules de polyaminoacides sélectionnés fait partie des éléments fondamentaux de la présente invention. Avantageusement, ces particules ont une taille moyenne comprise entre 0,01 et 200 µm, avec une répartition granulométrique étroite.

L'un des atouts de l'invention est d'être parvenue à un très bon contrôle de la granulométrie moyenne des particules et de leur répartition granulométrique. Ce contrôle passe par l'atteinte de tailles de particules extrêmement réduites, de l'ordre de quelques nanomètres et de très faible polydispersité, sachant qu'il est possible d'augmenter la taille de ces nanoparticules par agrégation. Sans que cela ne soit limitatif, on peut ainsi distinguer deux populations de particules en fonction de leurs tailles.

La première de ces populations regroupe les particules de type **NPV** nanoparticules, de taille moyenne comprise entre 0,01 µm et 0,5 µm, de préférence entre 0,03 et 0,4 µm.

La deuxième population comprend les particules de type **MPV**, de taille moyenne supérieure à 0,5 µm, de préférence inférieure ou égale à 20 µm.

Au sens de l'invention, on entend, par taille ou granulométrie moyenne, la moyenne arithmétique des diamètres en volume [D4,3] établis par diffraction laser dans le cas des **MPV**, et le diamètre de gyration mesuré par diffusion élastique de la lumière dans le cas des **NPV**.

Les *microparticules* **MPV** sont, avantageusement, obtenues à partir des *nanoparticules* **NPV**, e. g. par agrégation.

Selon une variante, les *microparticules* comprennent au moins un agent d'agrégation.

Conformément à une caractéristique préférée de l'invention, les particules comprennent au moins un principe actif.

Le contrôle de la taille des **MPV** et **NPV** s'opère, également, par l'intermédiaire de la composition des polyaminoacides mais aussi, pour une même composition, de la structure ordonnée (séquentielle alternée, i. e. en blocs : S₁) ou désordonnée (séquentielle aléatoire, i. e. statistique : S₂).

La nomenclature qui sera utilisée, dans le présent exposé, pour nommer les polyaminoacides est la suivante : poly AAN1/AAN2/.../AAI1/AAI2/...-A/B/C/D..., A, B, C, D... étant les pourcentages molaires des acides aminés. De plus, on distingue la structure ordonnée en blocs de la structure désordonnée ou statistique en ajoutant le terme "blocs". Par exemple, le copolymère statistique composé de 30 % de leucine et de 70 % d'acide glutamique est le poly Leu/Glu-30/70 et le copolymère avec la même composition et de structure blocs (leu)ₙ-(Glu)ₘ est le poly Leu/Glu blocs-30/70.

Suivant un mode préféré de réalisation de l'invention, les particules sont caractérisées en ce que AAI = Glu et AAN = Leu.

Outre les particules décrites supra à titre de produit nouveau *per se,* la présente invention a également pour objet un procédé de préparation de particules à base de polyaminoacide(s) et susceptibles d'être utilisées comme vecteurs de principe(s) actif(s), caractérisé :
- en ce que l'on met en oeuvre des polyaminoacides **(PAA)** :
   ▲ comprenant au moins deux types d'aminoacides récurrents AAN et AAI :
      . le type AAN correspondant à un acide aminé neutre hydrophobe,
      . et le type AAI correspondant à un acide aminé à chaîne latérale ionisable,
         les aminoacides récurrents de chaque type AAN et AAI étant identiques ou différents entre eux,

      ▲ le ratio molaire AAN / AAI + AAN étant ≥ 3 %, de préférence ≥ 5 %,
      ▲ la masse molaire en poids M_{w} du (ou des) polyaminoacide(s) étant supérieure ou égale à 4 000 D, de préférence à 5 000 D,

   - en ce que l'on réalise une dispersion de ces polyaminoacides dans un liquide, de préférence dans une solution aqueuse saline, dont on a ajusté le pH à une valeur choisie de telle sorte qu'au moins une partie des aminoacides de type AAI soit sous forme ionisée,
   - et en ce que l'on recueille ainsi une solution colloïdale de particules.

La description des caractéristiques des **PAA** faite supra, dans le cadre de la présentation des particules, peut être intégralement transposée dans le présent exposé relatif au procédé.
Ce procédé est l'un de ceux permettant d'obtenir les particules **NPV** présentées ci-avant. Ces particules peuvent donc être celles dans lesquelles l'AAN (ou les ANN) est(sont) choisi(s) dans la liste suivante : Leu - Ile - Val - Ala - Pro -Phe - et leurs mélanges et celles dans lesquelles l'AAI (ou les AAI) est(sont) formé(s) par le Glu et/ou l'Asp.

La formation des **NPV** se produit donc de manière simple, dans une solution aqueuse saline (par exemple) et à un pH choisi de telle sorte qu'au moins une partie des monomères AAI (de nature identique ou différente entre eux) soit sous forme ionisée. Cette génération spontanée de nanoparticules, par dispersion de copolyaminoacides en milieu salin, est remarquable de simplicité, d'économie et donc de faisabilité industrielle.

De plus, il est possible, par cette méthode, d'éviter les solvants organiques, généralement utilisés pour préparer ce type de particules et qui sont connus pour entraîner la dénaturation des protéines.

Les conditions d'obtention de ces **NPV** sont aisément maîtrisables par l'homme du métier.

La formation de **NPV** dépend, d'une part, de la nature de la solution aqueuse de dispersion et, d'autre part, des caractéristiques du polyaminoacide.

Les solutions aqueuses de dispersion des polyaminoacides doivent satisfaire certaines conditions de pH et de force ionique. On conçoit, en effet, facilement que la stabilité des nanoparticules de polymères, contenant des groupements ionisés, dépend de la force ionique. Quant au pH, il est bien sûr fonction de la nature des groupements ionisables, dont il fixe la fraction f d'ionisation. Ainsi, pour des groupements carboxyliques, f croît avec le pH.

En tout état de cause, l'un des avantages certains du procédé selon l'invention est de permettre la formation spontanée des **NPV** indépendamment du pH, dans un domaine étendu de pH compris entre 3 et 13, ce qui couvre largement le domaine des pH biologiques et ouvre ainsi un large champ d'applications.

Les **NPV** de polyaminoacides forment des solutions colloïdales.

Pour ces polyaminoacides, les caractéristiques discriminantes de la formation de **NPV** sont :
i) - la masse molaire,
2i) - la nature des acides aminés,
3i) - les proportions en acides aminés,
4i) - la présence d'enchaînements linéaires, de préférence α-peptidiques,
5i) - la répartition des acides aminés le long des chaînes de polymères, de façon régulière ou aléatoire, suivant des structures respectivement "blocs" ou "statistique".

Ces caractéristiques sont discutées ci-dessous.

Concernant l'influence de la masse molaire, on peut indiquer que la formation des **NPV** résulte de l'association des acides aminés entre les chaînes de polyaminoacides et que celle-ci s'opère différemment suivant la structure et la masse molaire du polymère.

Pour les polyaminoacides de structure "statistique", les polymères de masses molaires supérieures ou égales à 10 000 D, de préférence comprise entre 20 000 D et 500 000 D et, plus préférentiellement, comprises entre 20 000 D et 150 000 D, se dispersent facilement en solution aqueuse et forment des suspensions colloïdales de NPV stables. Dans les mêmes conditions, les polymères de plus faible masse molaire ne forment pas de suspensions colloïdales stables, une partie des particules précipitent et les **NPV**, maintenues en dispersion, sont peu diffusantes. L'association des chaînes de polymères en **NPV** est donc d'autant plus favorable que la masse molaire des polyaminoacides est élevée.

Dans le cas des polyaminoacides de structure "blocs", l'association interchaînes, entre des blocs d'acides aminés identiques, est favorisée, ce qui autorise l'utilisation de polymère de plus faible masse molaire que les polyaminoacides de structure "statistique". Les polymères de masses molaires supérieures ou égales à 5 000 D, de préférence comprise entre 6 500 D et 200 000 D et, plus préférentiellement, comprise entre 8 000 D et 20 000 D, se dispersent facilement en solution aqueuse et forment des suspensions colloïdales de **NPV** stables.

Concernant l'influence de la nature des acides aminés et de leur proportion, on peut préciser que, dans le cas des **PAA** de leucine et d'acide glutamique, la fraction en leucine doit être suffisamment élevée pour éviter que le polymère ne soit totalement soluble et assurer des interactions hydrophobes suffisantes pour que les chaînes de polymères s'associent en **NPV**. Ces intéractions interchaînes sont favorisées avec les polyaminoacides "blocs" et la fraction de leucine minimale nécessaire pour former des **NPV** est plus faible avec les polymères "blocs" qu'avec les polymères "statistiques". On a pu montrer, par exemple, que la concentration critique en dessous de laquelle le polymère est soluble, est comprise entre 20 et 30 % pour les polymères "statistiques" de leucine et d'acide glutamique.

Pour la mise en oeuvre de la préparation de **NPV** conformes à l'invention, on fixe, avantageusement, la molarité de la solution saline entre 10⁻⁴ et 1 M, de préférence 10⁻² M à 0,5 M environ.

Suivant une autre modalité pratique de l'invention, on choisit les concentrations en polymère dans la solution exprimées en % poids/volume, supérieures ou égales à 10⁻², de préférence comprises entre 0,05 et 30 et, plus préférentiellement encore, entre 0,05 et 5.

Dans la mesure où l'une des applications les plus remarquables des particules et leur procédé d'obtention selon l'invention est le transport sous protection de principes actifs dans l'organisme humain ou animal, il est avantageux de prévoir, à cette fin, qu'au moins un principe actif soit dissous dans le milieu liquide de formation des particules.

Cette mise en solution du principe actif, en particulier protéique et polypeptidique, s'effectue, de préférence, avant introduction des polyaminoacides dans le milieu, de sorte que l'on obtienne, après cette introduction, une solution colloïdale de particules chargées en principe actif.

Sans vouloir être lié par la théorie, on peut supposer que l'interaction entre le **PA** et les polyaminoacides procède d'associations hydrophobes et électrostatiques.

En résumé, l'encapsulation selon l'invention consiste donc à :
- mettre en solution aqueuse le **PA** à encapsuler,
- et à disperser du polyaminoacide dans une solution aqueuse, puis à mélanger la suspension colloïdale de nanoparticules ainsi formée à la solution de **PA**, ou bien, et de façon préférée, à disperser directement du polyaminoacide dans la solution de **PA**, de manière à obtenir spontanément des nanoparticules chargées en **PA**.

Une des caractéristiques essentielles majeures de l'invention est que le phénomène d'association du (ou des) **PA** avec les particules est indépendant du pH.

Il a été vu ci-dessus que la dispersion du copolyaminoacide dans le milieu liquide, salin de préférence, constitue une étape clé du procédé de préparation de particules éventuellement chargées en **PA** selon l'invention. Le procédé selon l'invention se singularise, également, en ce qu'il comprend au moins une étape supplémentaire d'agrégation des *nanoparticules* (**NPV**) en *microparticules* (**MPV**), de préférence à l'aide d'un sel et/ou d'un acide et/ou d'un polymère (avantageusement un polyélectrolyte).

Grâce à cette caractéristique du procédé de l'invention, il est possible d'agréger des **NPV** de taille comprise entre 0,01 et 0,05 µm, en **MPV** de taille comprise entre 0,05 et 200 µm, de préférence entre 0,05 et 20 µm et, plus idéalement encore, entre 0,05 et 10 µm.

Cette agrégation doit être réalisée dans des conditions non dénaturantes pour le **PA** et la Demanderesse a trouvé que l'addition, notamment de sels ou d'acides ou de polymères cationiques, entraîne l'agrégation des **NPV** en **MPV**.

L'addition de sels permet d'augmenter la force ionique du milieu et provoque l'agrégation des **NPV** en écrantant les répulsions électrostatiques entre les particules. De plus, le sel peut aussi agir comme agent de réticulation des fonctions carboxyliques des polyaminoacides présentes à la surface des particules et provoquer ainsi leur agrégation par complexation de plusieurs acides carboxyliques sur le cation du sel. Dans ce cas, on choisira, de préférence, des sels polycationiques parmi ceux formant des complexes avec les acides carboxyliques, tels que les sels de Fe²⁺, Fe³⁺, Zn²⁺, Ca²⁺, Al²⁺, Al³⁺ et Cu²⁺.

L'addition d'acides diminue la fraction f d'ionisation en neutralisant les fonctions carboxyliques des polyaminoacides et entraîne ainsi l'agrégation des **NPV** en **MPV**. La fraction d'ionisation à laquelle s'opère l'agrégation dépend de la composition du polyaminoacide AAN/(AAN + AAI). Elle est d'autant plus faible que la proportion en AAI est élevée. L'acide qui est additionné est, avantageusement, un acide fort de pKa inférieur à celui des fonctions carboxyliques dans les polyaminoacides.

Les polymères cationiques agissent comme des agents d'agrégation en associant les **NPV** : ils forment des complexes entre les fonctions carboxyliques à la surface des particules qui sont ainsi reliées entre elles par les molécules de polymères cationiques.

Les conditions d'agrégation des **NPV** en **MPV** sont développées dans les exemples.

En fin de procédé, avec ou sans encapsulation de **PA**, on récupère les (nano) et les (micro)particules par tout moyen connu en soi et approprié. En pratique on peut, par exemple, faire usage de la centrifugation et la lyophylisation.

Le principe actif, susceptible d'être inclus ou incorporé (de préférence selon une configuration de type matrice) dans les particules selon l'invention, obtenues ou non par le procédé décrit supra, est médicamenteux et/ou nutritionnel. Il est, de préférence, choisi parmi :
▲ les protéines et/ou les peptides parmi lesquels les plus préférentiellement retenus sont : les hémoglobines, les cytochromes, les albumines, les interférons, les antigènes, les anticorps, la calatonine, l'érythropoïétine, l'insuline, les hormones de croissance, le facteur IX, l'interleukine ou leurs mélanges,
▲ les polysaccharides, l'héparine étant plus particulièrement sélectionnée,
▲ les acides nucléiques et, préférablement, les oligonucléotides d'ARN et/ou d'ADN,
▲ et leurs mélanges.

Les **PA**, que l'on peut classer dans la catégorie des médicaments et qui sont propres à être vectorisés par les particules selon l'invention, sont les vaccins.

A titre d'exemple de produit nutritionnel, on peut citer les vitamines, les acides aminés et les oligoéléments.

Selon un autre de ses aspects, l'invention vise également l'utilisation de ces **NPV** et **MPV** chargées en **PA**, pour la fabrication de médicaments du type systèmes à libération contrôlée de **PA**.

La présente invention concerne, enfin, les médicaments ou les spécialités pharmaceutiques ou nutritionnelles comprenant les **PV** chargées en **PA** et définies ci-dessus.

Dans le cas de médicaments, il peut s'agir, par exemple, de ceux administrables, de préférence par voie orale, nasale, vaginale, oculaire, sous-cutanée, intraveineuse, intramusculaire, intradermique, intrapéritonéale, intracérébrale ou parentérale.

Les applications de l'invention ne sont pas limitées à la vectorisation, au transport de **PA** de nature médicamenteuse ou nutritionnelle. En effet, il est tout à fait concevable que le **PA**, susceptible d'être inclus ou incorporé dans les **PV**, soit au moins un produit cosmétique ou phytosanitaire. Les applications cosmétiques envisageables sont, par exemple, les compositions applicables par voies transdermiques. Les produits phytosanitaires concernés peuvent être, par exemple, des herbicides, des pesticides, des insecticides, des fongicides, etc.. La présente invention a également pour objet les compositions phytosanitaires et cosmétiques comprenant des **PV** chargées en **PA** du type de ceux visés supra.

Les exemples qui suivent permettront de mieux comprendre l'invention dans ses différents aspects produit/procédé/application. Ces exemples illustrent la prépration de particules de polyaminoacides chargés ou non en principes actifs, de même qu'ils présentent les caractéristiques de structure et les propriétés de ces particules.

### EXEMPLES

### I - PRÉPARATION DES POLYAMINOACIDES TESTÉS

Les polymères mis en oeuvre dans les exemples sont des copolymères synthétiques linéaires, de structures en blocs ou statistiques, à base de leucine et d'acide glutamique. Les polyaminoacides ont des masses molaires en poids M_{w}, déterminées par diffusion élastique de la lumière dans le solvant acide trifluoracétique, comprises entre 50 000 D et 150 000 D.

Ces polymères sont obtenus à partir du copolymère de leucine et de glutamate de méthyle dont on rétablit la fonctionnalité des chaînes latérales ionisables du glutamate de sodium, en utilisant les procédés de déprotection connus des esters méthyliques décrits, par exemple, par STAHMAN et coll., J. Biol. Chem., 197, 771 (1952) ou dans le brevet KYOWA HAKKO, FR 2 152 582.

Le copolymère de leucine et de glutamate de méthyle est obtenu à partir des anhydrides des N-carboxy-a-aminoacides **(NCA)** de leucine et de glutamate de méthyle, dont la préparation est donnée par exemple dans Biopolymers, 15, 1869 (1976). Les techniques utilisées, pour la polymérisation des **NCA** en polymères de structures en blocs ou statistiques sont connues de l'homme de l'art et sont détaillées dans l'ouvrage de H. R. KRICHELDORF "α-aminoacides-N-Carboxy Anhydrides and Related Heterocycles", Springer Verlag (1987).

### EXEMPLE 1 : SYNTHESE D'UN POLYAMINOACIDE "STATISTIQUE", LE POLY(LEU/GLU) 50/50.

### ETAPE 1): COPOLYMÉRISATION DES NCA- LEU ET NCA-GLU(OME) : POLY(LEU-CO-GLU(OME)) 50/50 :

Dans un réacteur de 1 1, muni d'un agitateur en verre, d'une arrivée d'azote et d'une sortie reliée à un bulleur, on introduit, sous courant d'azote, 15,0 g de N-carboxyanhydride de la glutamate de méthyle (NCA-Glu(OMe) : 0,08 mole) et 12,5 g de N-carboxyanhydride de la leucine (NCA-Leu : 0,08 mole). 381 ml de dioxane sont rajoutés et le milieu réactionnel est porté à 40 °C.
Après dissolution des NCA, on introduit 24 ml d'eau, suivi de 0,22 ml de triéthylamine (soit 1 % molaire par rapport aux NCA). Le suivi de la polymérisation est effectué par IR en observant la disparition des bandes carbonyles à 1 860 et 1 790 cm⁻¹. La durée de polymérisation varie entre 1,5 h et 3 h selon la composition des monomères. Après disparition totale des bandes, le milieu réactionnel est dilué par 380 ml de dioxane, puis homogénéisé pendant 3 h à température ambiante. Le copolymère est récupéré par précipitation dans 5 l d'eau sous bonne agitation. Le produit est filtré et séché à 50 °C sous vide pendant 12 h.
La masse de copolymère obtenu est de 18,4 g, soit un rendement pondéral de 90 %. RMN 1H (acide trifluoroacétique-d) : 0,85 ppm (CH₃-Leu, 6H∗0,5); 1,58 (CH₂ et CHMe₂ Leu, 3H∗0,5); 2,10 et 2,22 (CH₂-Glu, 2H*0,5) ; 2,58 (CH₂-Glu ; 2H*0,5); 3,75 (CH₃-Glu, 3H*0,5) ; 4,62 (NCHCO-Leu, 1H∗0,5) ; 4,70 (NCHCO-Glu, 1H∗0,5). Viscosité réduite (0,5 g/dl dans l'acide trifluoroacétique) à 25 °C = 2,2 dl/g.

### ETAPE 2): HYDROLYSE DE L'ESTER MÉTHYLIQUE DU POLY(LEU-CO-GLU(OME)) 50/50 :

Le copolymère obtenu précédemment (17,7 g) est placé dans un réacteur dans lequel on rajoute 354 ml d'acide trifluoroacétique. Le milieu réactionnel est porté à 40 °C sous agitation. Lorsque le copolymère est totalement dissous, on rajoute 354 ml d'eau par petites quantités. Le milieu réactionnel est maintenu sous agitation pendant 48 h.
Le polymère est récupéré par précipitation dans 5 1 d'eau. Après filtration, il est à nouveau mis en suspension et agité dans l'eau pendant 0,5 h, puis filtré et essoré. La purification s'effectue par dialyse dans l'eau.
Rendement 15,9g (95 %). RMN 1H (acide trifluoroacétique-d) : identique aux polymères de départ à une exception, le signal à 3,75 (CH₃-Glu) est fortement diminué ou absent. Dans le cas présent, le taux d'esters résiduels est inférieur à 1 % par rapport aux monomères glutamate. Viscosité réduite (0,5 g/dl dans l'acide trifluoroacétique) à 25 °C = 0,95 dl/g.

### EXEMPLE 2 : SYNTHÈSE D'UN POLVAMINOACIDE "BLOC", LE POLY(LEU/GLU) 50/50 DIBLOCS.

Dans un réacteur de 1 l, on introduit sous agitation 15,0 g de NCA-Glu(OMe) (0,08 mole) et 180 ml de dioxane. Après dissolution, on rajoute 180 ml de toluène et le milieu est porté à 60 °C. On effectue le spectre IR de la solution avant de rajouter 0,156 g de benzylamine (1,58 % molaire/NCA). Le milieu réactionnel se trouble rapidement et, au bout de 40 minutes, les bandes caractéristiques à 1 860 et 1 790 cm⁻¹ ont disparu.
Après une heure, on introduit une solution de 12,5 g de NCA-Leu (0,08 mole) dans un mélange dioxane/toluène (15 ml de chaque). L'agitation est poursuivie pendant 18 h (cette durée n'a pas été optimisée). Les bandes carbonyles ont alors disparu. On ajoute 100 ml de dioxane et le milieu réactionnel est homogénéisé pendant 1 h. Le copolymère est précipité dans 3 l d'éthanol absolu sous forte agitation. Il est lavé avec 1 l d'éthanol, filtré, essoré et enfin séché à 50 °C sous vide pendant une nuit. La masse de produit récupérée est de 19,5 g (rendement = 95 %). RMN 1H (acide trifluoroacétique-d) : 0,85 ppm (CH₃-Leu, 6H*0,5) ; 1,58 (CH₂ et CHMe₂ Leu, 3H*0,5) ; 2,10 et 2,22 (CH₂-Glu, 2H*0,5) ; 2.58 (CH₂-Glu ; 2H*0,5) ; 3,75 (CH₃-Glu, 3H*0,5) ; 4,62 (NCHCO-Leu, 1H*0,5) ; 4,70 (NCHCO-Glu, 1H*0,5). Viscosité réduite (0,5 g/dl dans l'acide trifluoroacétique) à 25 °C = 0,62 dl/g.

La deuxième étape d'hydrolyse des esters méthyliques est identique à celle décrite dans l'exemple 1, étape 2. Rendement 95 %. RMN 1H (acide trifluoroacétique-d): identique aux polymères de départ à une exception, le signal à 3,75 (CH₃-Glu) est fortement diminué ou absent. Dans le cas présent, le taux d'esters résiduels est inférieur à 1 % par rapport aux monomères glutamate. Viscosité réduite (0,5 g/dl dans l'acide trifluoroacétique) à 25 °C = 0,55 dl/g.

### EXEMPLE 3: SYNTHÈSE D'UN POLYAMINOACIDE "BLOC", LE POLY(GLU/LEU/GLU) 29/57/14 TRIBLOCS.

Dans un réacteur de 1 l, on introduit sous agitation 7,5 g de NCA-Glu(OMe) (0,04 mole) et 180 ml de dioxane. Après dissolution, on rajoute 180 ml de toluène et le milieu est porté à 60 °C. On effectue le spectre IR de la solution avant de rajouter 0,156 g de benzylamine.
Après la disparition totale du monomère, on introduit une solution de 12,5 g de NCA-Leu (0,08 mole) dans un mélange dioxane/toluène (15 ml de chaque). L'agitation est poursuivie pendant 18 h. Ensuite, on introduit à nouveau 7,5 g de NCA-Glu(OMe) (0,04 mole) qu'on permet de réagir pendant 12 heures. On ajoute 100 ml de dioxane et le milieu réactionnel est homogénéisé pendant 1 h. Le copolymère est précipité dans 3 l d'éthanol absolu sous forte agitation. Il est lavé avec 1 1 d'éthanol, filtré, essoré et enfin séché à 50 °C sous vide pendant une nuit. La masse de produit récupéré est de 19,4 g (rendement = 95 %). RMN 1H (acide trifluoroacétique-d) : 0,85 ppm (CH₃-Leu, 6H*0,5) ; 1,58 (CH₂ et CHMe₂ Leu, 3H*0,5) ; 2,10 et 2,22 (CH₂-Glu, 2H*0,37) ; 2,58 (CH₂-Glu; 2H*0,37) ; 3,75 (CH₃-Glu, 3H*0,37) ; 4,62 (NCHCO-Leu, 1H*0,5) ; 4,70 (NCHCO-Glu, 1H*0,37). Viscosité réduite (0,5 g/dl dans l'acide trifluoroacétique) à 25 °C = 0,58 dl/g.

La deuxième étape d'hydrolyse des esters méthyliques est identique à celle décrite dans l'exemple 1, étape 2. RMN 1H (acide trifluoroacétique-d) : identique aux polymère de départ à une exception, le signal à 3,75 (CH₃-Glu) est fortement diminué ou absent. Dans le cas présent, le taux d'esters résiduels est inférieur à 1 % par rapport aux monomères glutamate. Viscosité réduite (0,5 g/dl dans l'acide trifluoroacétique) à 25 °C = 0,38 dl/g.

### II - FORMATION DE NANOPARTICULES DE POLYAMINOACIDES (NPV) AVEC OU SANS INCORPORATION DE PRINCIPES ACTIFS

### II.1 - INFLUENCE DE LA CONCENTRATION EN AAN SUR LA FORMATION DE PARTICULES

### EXEMPLE 4 : FORMATION DE NANOPARTICULES DE POLY(LEU/GLU) 30/70, 50/50, 75/25 DE STRUCTURE "STATISTIQUE".

100 mg de copolyaminoacides statistiques de leucine et de glutamate de sodium, de composition Leu/Glu = 30/70 et de masse molaire M_{w} = 36 000 D, sont dispersés dans 100 ml d'une solution de chlorure de sodium de molarité 10⁻² mol/l. Quel que soit le pH de la solution compris entre 4,5 et 12 que l'on peut ajuster par addition d'acide chlorhydrique ou d'hydroxyde de sodium, le polymère forme, spontanément, une dispersion colloïdale de nanoparticules. En milieu acide de pH inférieur à 4,5, qui correspond à une fraction d'ionisation f égale à 0,05, le polymère lyophilisé ne se disperse pas dans la solution et reste insoluble.
Le tableau 1 ci-dessous rassemble les observations réalisées dans les mêmes conditions de dispersion, avec les copolyaminoacides statistiques de leucine et de glutamate de sodium, de composition Leu/Glu = 50/50 et 75/25 et de masse molaire M_{w} égales, respectivement, à 60 000 D et 34 000 D.

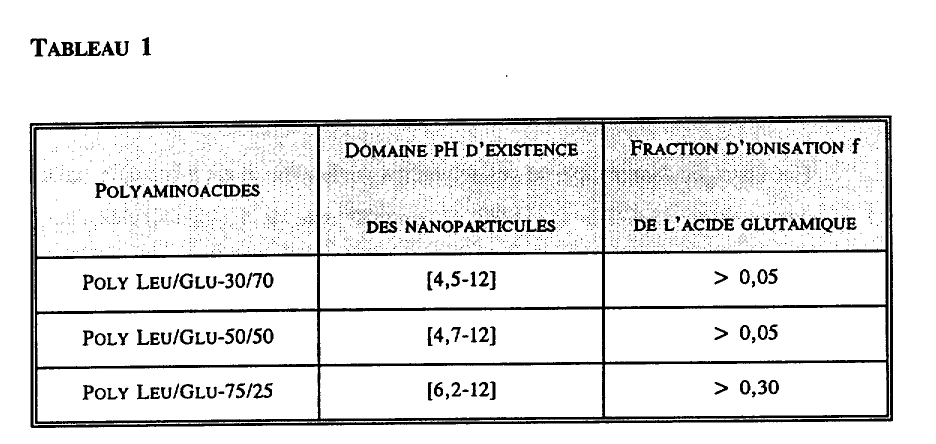

### EXEMPLE 5: FORMATION DE NANOPARTICULES DE POLY(LEU/GLU) 20/80, 40/60, 50/50 DE STRUCTURES "BLOCS".

100 mg de copolyaminoacides blocs de leucine et de glutamate de sodium, de composition Leu/Glu = 50/50 et de masse molaire M_{w} = 14 600 D, sont dispersés dans 100 ml d'une solution de chlorure de sodium de molarité 10⁻² mol/l. Quel que soit le pH de la solution, compris entre 3 et 12, que l'on peut ajuster par addition d'acide chlorhydrique ou d'hydroxyde de sodium, le polymère forme spontanément une dispersion colloïdale de nanoparticules qui diffusent la lumière et confèrent à la solution une turbidité élevée. Les nanoparticules de polymère ne décantent pas lorsque la solution est laissée plusieurs heures au repos à température ambiante comprise entre 15 et 20 ° C. En milieu acide de pH inférieur à 3, le polymère ne se disperse pas dans la solution et reste insoluble.
Dans les mêmes conditions à pH compris entre 3 et 12, les poly(Leu/Glu) 20/80, 40/60 de structures "blocs", de masse molaire M_{w} respectivement égales à 11 000 D, 15 000 D, se dispersent et forment des suspensions colloïdales. Ces suspensions colloïdales diffusent d'autant plus la lumière que la proportion de leucine dans le polymère est élevée. A pH inférieur à 3, on observe, de la même façon que pour le poly(Leu/Glu) 50/50, que les polymères ne se dispersent pas et restent insolubles.

### EXEMPLE 6: SOLUBILITÉ DU POLY(LEU/GLU) 18/82 DE STRUCTURE "STATISTIQUE".

Cet exemple montre que les copolymères de leucine et de glutamate de sodium de composition Leu/Glu = 18/82 ne forment pas de nanoparticules, car ils sont totalement solubles dans l'eau, quel que soit le pH ≤ 4,5.
10 mg de poly Leu/Glu-18/82 lyophilisé sont dispersés dans 0,5 ml d'une solution de chlorure de sodium de molarité 10⁻² mol/l. Le polymère est totalement dissous et la solution est limpide.
Il ne se forme pas de nanoparticules.

### EXEMPLE 7: STABILITÉ DES SUSPENSIONS COLLOÏDALES DE DIFFÉRENTS POLY(LEU/GLU).

100 mg des copolyaminoacides statistiques de leucine et de glutamate de sodium de composition Leu/Glu = 30/70, 50/50, 75/25 et de masse molaire M_{w}, égale, respectivement, à 36 000 D, 60 000 D et 34 000 D, sont dispersés dans 10 ml, 5 ml et 2 ml d'une solution d'hydroxyde de sodium de molarité 10⁻² mol/l, formant ainsi des solutions de concentration 1 %, 2 % et 5 % p/v de chacun des polyaminoacides. Les dispersions sont ensuite laissées en test de stabilité à température ambiante (15-25 °C) pendant 4 mois. A l'issue de cette période, les nanoparticules n'ont pas décanté et la diffusivité des solutions n'a pas changé.

### EXEMPLE 8 : FORMATION DE NANOPARTICULES DE POLY(LEU/GLU) 50/50 DE STRUCTURE "STATISTIQUE" ET DE STRUCTURE "BLOCS", DANS UN TAMPON PHOSPHATE ISOTONIQUE.

100 mg de polyaminoacides poly(Leu/Glu) 50/50, de structure "statistique" et de masse molaire M_{w} égale à 60 000 D, sont dispersés dans une solution de pH = 7,4 isotonique, contenant 0,01 mol/l de tampon phosphate, 0,138 mol/l de chlorure de sodium et 0,0027 mol/l de chlorure de potassium (PBS, voir catalogue SIGMA P4417). Le polymère forme spontanément une dispersion colloïdale de nanoparticules qui diffuse la lumière. Les nanoparticules de polymère ne décantent pas lorsque la solution est laissée plusieurs heures au repos à température ambiante entre 15 et 20 °C.
Dans les mêmes conditions, lorsque l'on disperse 100 mg de polyaminoacides poly(Leu/Glu) 50/50 blocs et de masse molaire M_{w} égale à 14 600 D, dans une solution tampon PBS, on obtient une suspension stable à température ambiante de nanoparticules qui diffuse la lumière avec une turbidité élevée.

### II.2 - DIMENSIONS ET STRUCTURE DES NANOPARTICULES DE POLY(LEU/GLU) DE STRUCTURE "STATISTIQUE" ET DE STRUCTURE "BLOC"

Les nanoparticules de polyaminoacides forment une solution colloïdale. Les mesures par diffusion statique ou quasi élastique de la lumière permettent de mesurer la taille et la densité de polymère dans les nanoparticules.
Le tableau 2 ci-dessous regroupe des mesures effectuées à partir des polyaminoacides statistiques de compositions Leu/Glu = 30/70, 50/50 et de masses molaires Mw comprises entre 46 000 D et 21 000 D, ainsi que du polyaminoacide "bloc" de compositions Leu/Glu 20/80, 50/50 de masses molaires comprises entre 11 000 D et 16 300 D. Pour ces mesures, les polyaminoacides sont dispersés dans une solution de pH = 7,4 isotonique, contenant 0,01 mol/l de tampon phosphate, 0,138 mol/l de chlorure de sodium et 0,0027 mol/l de chlorure de potassium (voir catalogue SIGMA P4417).

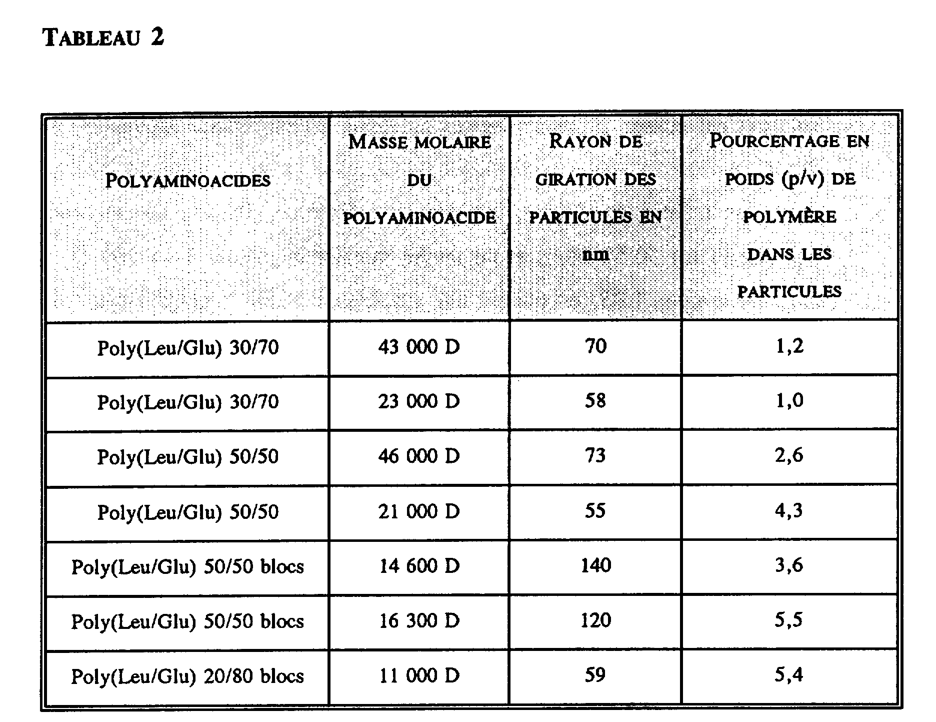

Les dimensions des nanoparticules varient avec la composition des polyaminoacides. Pour une même composition, elles dépendent de la structure dibloc ou désordonnée des chaînes de polyaminoacides.
De plus, les répartitions des diamètres des nanoparticules de polyaminoacides sont, d'après les analyses par diffusion quasi élastique de la lumière, monomodales et resserrées autour de leur valeur moyenne. Les largeurs des distributions obtenues sont comparables ou inférieures à celle du polystyrène de polydispersité égale à 1,2. La concentration de polymère dans les nanoparticules est remarquablement faible et toujours inférieure à 6 % p/v. Elle dépend de la composition et de la structure dibloc ou désordonnée des polyaminoacides.
Par ailleurs, l'observation par microscopie électronique (TEM à coloration négative) montre que les nanoparticules ont une forme sphérique ou légèrement allongée.

### II.3 - IMMUNOGÉNICITÉ DES NANOPARTICULES

### EXEMPLE 9 POUVOIR IMMUNOGENE DES NANOPARTICULES DE POLY(LEU/GLU) 40/60, 50/50, 60/40 BLOCS.

Les poly(Leu/Glu) 40/60, 50/50, 60/40 blocs, de masses molaires égales environ à 12 000 D, sont dispersés dans une solution de pH = 7,4 isotonique, contenant 0,01 mol/l de tampon phosphate, 0,138 mol/l de chlorure de sodium et 0,0027 mol/l de chlorure de potassium (voir catalogue SIGMA P4417). La concentration en polymère est égale à 2,5 mg/ml. Les suspensions sont turbides et filtrées sans difficulté particulière sur une membrane de polysulfone de porosité 0,2 µm, afin de les stériliser.
Les animaux utilisés sont des souris (lot de cinq souris par polymère testé) de souche non consanguine OF1.
Les suspensions de polymères sont injectées par voie sous-cutanée, à hauteur de 100 µl de suspension (250 µg de polymère) par injection. Une première injection est réalisée au temps J0, un rappel est effectué au temps J35. Le prélèvement est effectué au temps J42, soit 7 jours après la deuxième injection. Les prélèvements sanguins sont laissés 24 heures à température ambiante, puis sont centrifugés 10 min à 3 000 tr/min.
Les sérums sont anlysés par dosage ELISA. Aucun anticorps anti-polymère n'a été détecté dans les sérums, y compris à des faibles dilutions en sérum (1/10). Cet exemple montre que les nanoparticules de poly(Leu/Glu) 40/60, 50/50, 60/40 blocs n'induisent pas de réponse immunitaire spécifique.

### II.4 - ASSOCIATION DES NANOPARTICULES AVEC LES PROTÉINES MODÈLES COLOREES

Le procédé d'encapsulation est décrit avec, pour protéines modèles, l'hémoglobine, les cytochromes C de coeur de cheval et de *Saccharomyces cerevisea.* L'association entre les nanoparticules de polymère et les protéines est mise en évidence par ultracentrifugation analytique. Les solutions de polymères et protéines sont centrifugées à grandes vitesses et l'avancement des fronts de sédimentation du polymère et des protéines est suivi par la mesure de la densité optique aux longueurs d'onde à 250 nm et à 410 nm.
L'association entre les protéines et les particules colloïdales est caractérisée par l'existence d'un front unique de sédimentation correspondant à la superposition des fronts de sédimentation aux deux longueurs d'onde. Dans le cas contraire, en absence d'association, les fronts de sédimentation de la protéine et des particules colloïdales sont distincts et ne se superposent pas.

### EXEMPLE 10 : ASSOCIATION DU POLY(LEU/GLU) 30/70 AVEC LE CYTOCHROME C.

10 mg de cytochrome C sont dissous dans 100 ml d'une solution tampon de phosphate de sodium de pH égal à 7,2 et de molarité 0,01 mol/l. 100 mg de poly Leu/Glu 30/70, de masse molaire M_{w} = 36 000 D, sont ensuite dispersés directement dans cette solution. La plupart du cytochrome C sédimente avec les particules colloïdales de polymère lors de la centrifugation. L'analyse de la densité optique des fronts de sédimentation montre que 80 % du cytochrome sont associés aux particules colloïdales.

### EXEMPLE 11 : ASSOCIATION DU POLY(LEU/GLU) 50/50 AVEC LE CYTOCHROME C.

10 mg de cytochrome C sont dissous dans 100 ml d'une solution tampon de phosphate de sodium de pH égal à 7,2 et de molarité 0,01 mol/l. 200 mg de poly Leu/Glu-50/50, de masse molaire M_{w} = 60 000 D, sont ensuite dispersés directement dans cette solution. La plupart du cytochrome C sédimente avec les particules colloïdales de polymère lors de la centrifugation. L'analyse de la densité optique des fronts de sédimentation montre que plus de 80 % du cytochrome sont associés aux particules colloïdales.

### EXEMPLE 12 : ASSOCIATION DU POLY (LEU/GLU) 30/70 AVEC L'HÉMOGLOBINE.

Dans cet exemple, la suspension colloïdale du polyaminoacides et de la protéine est préparée de deux façons différentes, à partir de la même solution que celle de l'exemple 4, mais en modifiant l'ordre de mise en solution.
**1 -** Le poly Leu/Glu-30/70, de masse molaire M_{w} = 90 000 D, est dispersé dans la solution d'hémoglobine suivant les mêmes conditions que celles de l'exemple 4. L'analyse par ultracentrifugation de la suspension colloïdale montre l'association de l'hémoglobine et du polyaminoacides dans les nanoparticules.
**2 -** Le poly Leu/Glu-30/70, de masse molaire M_{w} = 40 000 D, est dispersé dans la solution tampon ne contenant pas d'hémoglobine. La suspension colloïdale formée est ensuite mélangée avec la solution d'hémoglobine. Dans ce cas, une fraction importante de l'hémoglobine, estimée à 80 %, n'est pas associée aux nanoparticules de polyaminoacides et l'analyse par ultracentrifugation montre deux fronts de sédimentation correspondant, respectivement, aux nanoparticules de polyaminoacides et à l'hémoglobine.

La première étape de mise en solution de la protéine, avant dispersion du polyaminoacide, assure, dans le cas de l'hémoglobine, un meilleur rendement d'encapsulation.

### II. 5 - ASSOCIATION DES NANOPARTICULES AVEC LES PROTÉINES

### EXEMPLE 13 : ASSOCIATION DU POLY(LEU/GLU) 30/70 EN PRÉSENCE D'OVALBUMINE.

Le poly Leu/Glu-30/70, de masse molaire M_{w} = 90 000 D, est dispersé dans une solution de chlorure de sodium dans les mêmes conditions que celles de l'exemple 2, 4 ou 5 avec, en plus, de l'ovalbumine. Les caractéristiques des particules colloïdales analysées par diffusion de la lumière sont identiques à celles formées en absence de protéine. La protéine n'empêche donc pas l'association des polyaminoacides en nanoparticules et ceci pour des concentrations en protéine pouvant atteindre 20 % p/p par rapport au polyaminoacide.

### EXEMPLE 14 : ASSOCIATION DU POLY(LEU/GLU) 50/50 BLOCS AVEC L'INSULINE.

A partir d'une solution isotonique de pH = 7,4 contenant 0,01 mol/l de tampon phosphate, 0,138 mol/l de chlorure de sodium et 0,0027 mol/l de chlorure de potassium, on prépare une solution d'insuline humaine recombinante (SIGMA, référence 10259) de concentration 1 mg/ml. Dans 5 ml de cette solution d'insuline, on disperse 50 mg de poly(Leu/Glu) 50/50 blocs de masse molaire égale à 12 400 D. On obtient une suspension très turbide et stable. Par ultra-filtration sur membrane de cut-off 300 000 D (Millipore, filtre Ultrafree-CI.), on sépare l'insuline libre en solution que l'on dose dans le filtrat par chromatographie HPLC, de l'insuline associée avec les nanoparticules. On mesure ainsi, par différence avec la quantité d'insuline libre, la quantité d'insuline associée aux nanoparticules égale à 0,65 mg/ml.

### EXEMPLE 15: ASSOCIATION DU POLY(LEU/GLU) 50/50 DE STRUCTURE "STATISTIQUE" AVEC L'INSULINE.

On procède dans des conditions identiques à celles de l'exemple 14 en utilisant le poly(Leu/Glu) 50/50 de structure "statistique" à la place du poly(Leu/Glu) 50/50 blocs. La quantité d'insuline, associée aux nanoparticules, est égale à 0,60 mg/ml.

### III - AGRÉGATION DES NANOPARTICULES

### III. I - AGRÉGATION PAR ADDITION D'UN SEL

### EXEMPLE 16: AGRÉGATION PAR ADDITION DE SULFATE D'AMMONIUM.

100 mg de poly Leu/Glu-30/70, de masse molaire M_{w} = 36 000 D, sont dispersés dans 200 ml de solution tampon d'acide citrique et de phosphate de sodium de molarité 0,05 mol/l et de pH égal à 5. Une solution concentrée de sulfate d'ammonium est additionnée lentement à la dispersion. Le volume versé est suffisamment faible devant celui de la solution de dispersion, jusqu'à agrégation des **NPV** en **MPV**. Les **MPV** ainsi obtenus ont un diamètre moyen égal à 8 µm.

### III.2 - AGRÉGATION PAR ABAISSEMENT DE pH

Les fonctions carboxyliques latérales des polyaminoacides dans les nanoparticules sont partiellement ionisées. Leur neutralisation, par addition d'un acide, entraîne l'agrégation des nanoparticules.
L'agrégation peut être réalisée avec les acides dont la constante de dissociation (Ka) est inférieure à celle des fonctions carboxyliques latérales des polyaminoacides.

### EXEMPLE 17: AGRÉGATION PAR ADDITION D'ACIDE CHLORHYDRIQUE.

Les polyaminoacides statistiques de composition Leu/Glu = 30/70, 50/50 et 75/25 et de masses molaires M_{w}, égales respectivement à 36 000 D, 60 000 D et 34 000 D, sont dispersés dans des solutions tampons d'acide citrique et de phosphate de sodium de molarité 0,05 mol/l et de pH égal à 5. Les concentrations des polyaminoacides sont de 0,01 % p/v pour les polyaminoacides de composition Leu/Glu-30/70 et 50/50 et 0,005 % p/v pour celui de composition 75/25.
L'agrégation des nanoparticules en suspension colloïdale est réalisée par addition progressive d'une solution 0,1 mol/l d'acide chlorhydrique, jusqu'à agrégation des **NPV** en **MPV**. Les résultats des mesures de granulométrie des MPV sont regroupés dans le tableau 3 suivant.

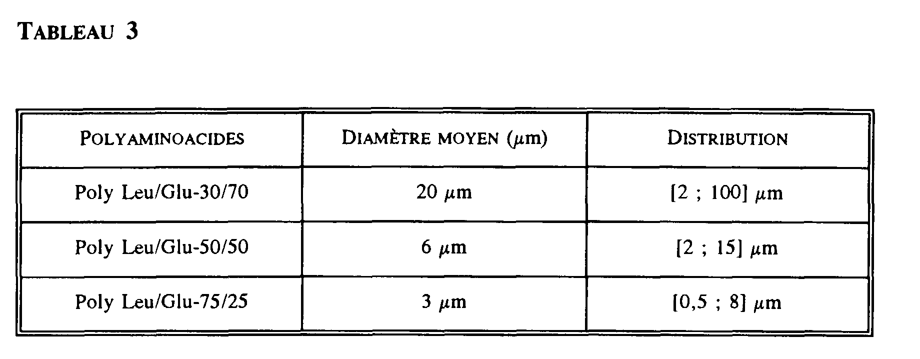

### III.3 - AGRÉGATION PAR COMPLEXATION AVEC UN POLYMÈRE CATIONIQUE

### EXEMPLE 18 : AGRÉGATION DES NANOPARTICULES DE POLY Leu/Glu-50/50 PAR COMPLEXATION AVEC LA POLY D,L-LYSINE.

Les fonctions carboxyliques latérales des polyaminoacides dans les nanoparticules sont partiellement ionisées. Leur complexation avec un polymère cationique, tel que la polyLysine, entraîne l'agrégation des nanoparticules.
10 mg de poly Leu/Glu-50/50, de masse molaire M_{w} = 60 000 D, sont dispersés dans 100 ml d'une solution tampon de phosphate de sodium de molarité 0,01 mol/l et de pH égal à 6. L'addition de 15 mg de bromure d'hydrogène de poly D,L-Lysine, de masse molaire M_{w} = 15 000 D, permet d'agréger les nanoparticules de polymère en microparticules. Le diamètre moyen des microparticules est compris entre 10 et 20 µm en faisant varier le pH entre 2 et 9 par addition d'acide chlorhydrique ou d'hydroxyde de sodium.

### III.4 - ENCAPSULATION DE PROTÉINES PAR AGRÉGATION DES NANOPARTICULES

### EXEMPLE 19: ENCAPSULATION DU CYTOCHROME C PAR COMPLEXATION AVEC LA POLY D,L-LYSINE.

Dans 100 ml d'une solution tampon de phosphate de molarité 0,01 mol/l, de pH égal à 6 et contenant 10 mg de cytochrome C de coeur de cheval, on disperse 10 mg de poly(Leu/Glu) 50/50, de masse molaire M_{w} = 60 000 D. L'addition de 15 mg de bromure d'hydrogène de poly D,L lysine, de masse molaire M_{w} = 15 000 D, permet d'agréger les nanoparticules de polymères en microparticules. Par centrifugation, les microparticules sont sédimentées ; la coloration rouge du culot de centrifugation montre que la quasi totalité du cytochrome a sédimenté avec les nanoparticules, ce qui montre que le cytochrome est encapsulé dans les microparticules.

## Revendications

1. Nanoparticules de vectorisation (NPV) de principe(s) actif(s), du type de celles à base de polyaminoacide(s),
caractérisées :
- en ce qu'elles ont une taille moyenne comprise entre 0,01 et 0,5 µm, de préférence entre 0,03 et 0,4 µm,
- en ce que ces NPV sont obtenues par mise en contact de polyaminoacides (**PAA**) avec une solution aqueuse,
- en ce que ces **PAA** sont linéaires à enchaînements α-peptidiques et comprennent au moins deux types d'aminoacides récurrents AAN et AAI :
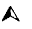 le type AAN correspondant à un acide aminé neutre hydrophobe,
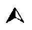 et le type AAI correspondant à un acide aminé à chaîne latérale ionisable, au moins une partie des aminoacides récurrents de type AAI étant sous forme ionisée,
les aminoacides récurrents de chaque type AAN et AAI étant identiques ou différents entre eux,
- en ce que la masse molaire en poids Mw des **PAA** est supérieure ou égale à 4 000 D, de préférence à 5000 D,
- et en ce que ces **PAA** sont non-hydrosolubles à pH acide et à pH compris entre 3 et 13.

2. Particules selon la revendication 1, caractérisées :
- en ce que leurs **PAA** constitutifs sont des **PAA** "blocs" et/ou des **PAA** "statistiques",
- et en ce que :
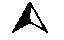 pour les **PAA** "blocs" :
. le rapport molaire AAN / AAN + AAI est ≥ 6 %, de préférence ≥ 15 %,
. M_{w} ≥ 5 500 D, de préférence 6 500 D ≤ M_{w} ≤ 200 000 D et, plus préférentiellement encore, 8 000 D ≤ M_{w} ≤ 200 000 D,
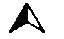 pour les **PAA** "statistiques" :
. le rapport molaire AAN / AAN + AAI est ≥ 20 %, de préférence ≥ 25 %,
. M_{w} ≥ 10 000 D, de préférence 20 000 D ≤ M_{w} ≤ 500 000 D et, plus préférentiellement encore, 200 000 D ≤ M_{w} ≤ 150 000 D.

3. Particules selon la revendication 1 ou 2, caractérisées :
- en ce que l'AAN (ou les AAN) est(sont)choisi(s) dans la liste suivante : Leu - Ile - Val - Ala - Pro - Phe - et leurs mélanges,
- et en ce que l'AAI (ou les AAI) est(sont) formé(s) par le Glu et/ou l'Asp.

4. Particules selon la revendication 1 ou 2, caractérisées en ce que le PAA comprend un aminoacide récurrent AAN et un aminoacide récurrent AAI.

5. Particules selon la revendication 1, caractérisées par une concentration moyenne en PAA variant de 0,01 à 25 % poids sec, de préférence de 0,05 à 10 % poids sec.

6. Particules selon la revendication 1, caractérisées en ce que ce sont des MicroParticules de Vectorisation (**MPV**) de taille moyenne supérieure à 0,5 µm, de préférence inférieure ou égale à 20 µm.

7. Particules selon la revendication 6, caractérisées en ce qu'elles sont obtenues à partir des particules selon la revendication 5 et en ce qu'elles comprennent, de préférence, au moins un agent d'agrégation.

8. Particules selon la revendication 1, caractérisées en ce qu'elles comprennent au moins un principe actif.

9. Procédé de préparation de particules à base de polyaminoacide(s) et susceptibles d'être utilisées comme vecteurs de principe(s) actif(s), caractérisé:
- en ce que l'on met en oeuvre des polyaminoacides (**PAA**):
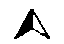 linéaires à enchaînement α-peptidiques,
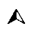 comprenant au moins deux types d'aminoacides récurrents AAN et AAI :
. le type AAN correspondant à un acide aminé neutre hydrophobe,
. et le type AAI correspondant à un acide aminé à chaîne latérale ionisable,
les aminoacides récurrents de chaque type AAN et AAI étant identiques ou différents entre eux,
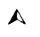 le ratio molaire AAN / AAI + AAN étant ≥ 3 %, de préférence ≥ 5 % ,
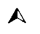 la masse molaire en poids M_{w} du (ou des) polyaminoacide(s) étant supérieure ou égale à 4 000 D, de préférence à 5 000 D,
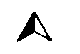 lesdits **PAA** étant non hydrosolubles à pH acide et à pH compris entre 3 et 13,
- en ce que l'on réalise une dispersion de ces polyaminoacides dans un liquide, de préférence dans une solution aqueuse,
- et en ce que l'on recueille ainsi une solution colloïdale de particules.

10. Procédé selon la revendication 9, caractérisé en ce que l'on met en oeuvre un **PAA** comprenant un aminoacide récurrent AAN et un aminoacide récurrent AAI.

11. Procédé selon la revendication 9, caractérisé en ce qu'au moins un principe actif est dissous dans le liquide, de préférence avant introduction des polyaminoacides dans le milieu, de sorte que l'on obtienne, après cette introduction, une solution colloïdale de particules chargées en principe(s) actif(s).

12. Procédé selon la revendication 9, caractérisé en ce qu'il comprend au moins une étape supplémentaire d'agrégation des particules, à l'aide d'au moins un agent d'agrégation constitué, de préférence, par un sel et/ou un acide et/ou une base et/ou un polymère éventuellement ionique.

13. Procédé selon la revendication 12, caractérisé en ce que l'on choisit des concentrations en polymère dans la solution exprimées en % poids/volume, supérieures ou égales à 10⁻², de préférence comprises entre 0,05 et 30 et, plus préférentiellement encore, entre 0,05 et 5.

14. Particules selon la revendication 1 et/ou obtenues par le procédé selon la revendication 9,
caractérisées en ce que le principe actif est médicamenteux et, de préférence, choisi parmi:
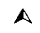 les protéines et/ou les peptides parmi lesquels les plus préférentiellement retenus sont : les hémoglobines, les cytochromes, les albumines, les interférons, les antigènes, les anticorps, la calatonine, l'érythropoïétine, l'insuline, les hormones de croissance, le facteur IX, l'interleukine ou leurs mélanges,
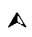 les polysaccharides, l'héparine étant plus particulièrement sélectionnée,
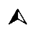 les acides nucléiques et, préférablement, les oligonucléotides d'ARN et/ou d'ADN,
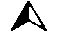 et leurs mélanges.

15. Particules selon la revendication 1 et/ou obtenues par le procédé selon la revendication 9, caractérisées en ce que le principe actif est constitué par au moins un vaccin.

16. Particules selon la revendication 1 et/ou obtenues par le procédé selon la revendication 9, caractérisées en ce que le **PA** est formé par au moins un produit phytosanitaire ou cosmétique.

17. Spécialité pharmaceutique, nutritionnelle, phytosanitaire ou cosmétique,
caractérisée en ce qu'elle comporte des particules selon la revendication 1 et/ou obtenues par le procédé selon la revendication 9.

## Patentansprüche

1. Nanopartikel-Träger (NPV) für Wirkstoff(e) auf Polyaminosäure(n)basis,
**dadurch gekennzeichnet, daß**
- ihre durchschnittliche Größe zwischen 0,01 und 0,5 µm, vorzugsweise zwischen 0,03 und 0,4 um beträgt,
- diese NPV hergestellt werden, indem Polyaminosäuren (PAA) mit einer wässerigen Lösung in Kontakt gebracht werden,
- diese PAA linear durch α-Peptidbindungen gebildet sind und mindestens zwei Typen von rekurrenten Aminosäuren, nämlich AAN und AAI enthalten:
• wobei der Typ AAN einer neutralen, wasserabstoßenden Aminosäure entspricht, und
• der Typ AAI entspricht einer Aminosäure mit ionisierbarer Seitenkette, wobei mindestens ein Teil der rekurrenten Aminosäuren des Typs AAI in ionisierter Form besteht, wobei die rekurrenten Aminosäuren der Typen AAN und AAI identisch oder voneinander unterschieden sind,
- die Molmasse M_{w} der PAA größer oder gleich 4 000 D, vorzugsweise 5000 D ist, und
- diese PAA bei einem im Säurebereich liegenden pH-Wert und bei einem pH-Wert von 3 bis 13 nicht wasserlöslich sind.

2. Partikel nach Anspruch 1, **dadurch gekennzeichnet, daß** ihre PAA-Bestandteile "Block"-PAA und/oder "statistische" PAA sind, und daß
- bei den "Block""-PAA
• das Molverhältnis AAN / AAN + AAI ≥ 6 %, vorzugsweise ≥ 15 % ist,
• M_{w} ≥ 5 500 D, vorzugsweise 6500 D ≤ M_{w} ≤ 200 000 D und noch besser 8000 D ≤ M_{w} ≤ 200 000 D ist,
- bei den "statistischen" PAA
• das Molverhältnis AAN / AAN + AAI ≥ 20 %, vorzugsweise ≥ 25 % ist,
• M_{w} ≥ 10 000 D, vorzugsweise 20 000 D ≤ M_{w} ≤ 500 000 D und noch besser 20 000 D ≤ M_{w} ≤ 150 000 D ist.

3. Partikel nach Anspruch 1 oder 2, **dadurch gekennzeichnet, daß**
- die AAN aus folgender Liste gewählt wird (werden): Leu - Ile - Val - Ala - Pro - Phe - und deren Gemische, und
- die AAI aus Glu und/oder Asp besteht (bestehen).

4. Partikel nach Anspruch 1 oder 2, **dadurch gekennzeichnet, daß** die PAA eine rekurrente Aminosäure des Typs AAN und eine *rekurrente* Aminosäure des Typs AAI enthält.

5. Partikel nach Anspruch 1, **dadurch gekennzeichnet, daß** ihre durchschnittliche PAA-Konzentration sich in einem Bereich von 0,01 bis 25 % der Trockenmasse, vorzugsweise von 0,05 bis 10 % der Trockenmasse bewegt.

6. Partikel nach Anspruch 1, **dadurch gekennzeichnet, daß** es sich um Mikropartikel-Träger (MPV) handelt, deren durchschnittliche Größe über 0.5 um beträgt und vorzugsweise kleiner oder gleich 20 um ist.

7. Partikel nach Anspruch 6, **dadurch gekennzeichnet, daß** sie aus Partikeln gemäß Anspruch 5 hergestellt werden und vorzugsweise mindestens ein Aggregationsmittel enthalten.

8. Partikel nach Anspruch 1, **dadurch gekennzeichnet, daß** sie mindestens einen Wirkstoff enthalten.

9. Verfahren zur Herstellung von Partikeln auf Polyaminosäure(n)basis, die als Wirkstoffträger verwendbar sind, **dadurch gekennzeichnet, daß**
- Polyaminosäuren (PAA) eingesetzt werden, die
• linear durch α-Peptidbindungen gebildet sind
• mindestens zwei Typen von rekurrenten Aminosäuren, nämlich AAN und AAI enthalten, wobei:
- der Typ AAN einer neutralen, wasserabstoßenden Aminosäure entspricht, und
- der Typ AAI einer Aminosäure mit ionisierbarer Seitenkette entspricht,
wobei die rekurrenten Aminosäuren der Typen AAN und AAI identisch oder voneinander unterschieden sind,
• das Molverhältnis AAN / AAN + AAI ≥ 3 %, vorzugsweise ≥ 5 % ist,
• die Molmasse M_{w} der Polyaminsäure(n) größer oder gleich 4 000 D, vorzugsweise 5 000 D ist, und
• diese PAA bei einem im Säurebereich liegenden pH-Wert und bei einem pH-Wert von 3 bis 13 nicht wasserlöslich sind,
- daß eine Dispersion dieser Polyaminsäuren in einer Flüssigkeit, vorzugsweise in einer wässerigen Flüssigkeit hergestellt wird, und
- daß auf diese Weise eine Partikel-Kolloidlösung gewonnen wird.

10. Verfahren nach Anspruch 9, **dadurch gekennzeichnet, daß** eine PAA eingesetzt wird , die eine rekurrente Aminosäure des Typs AAN und eine rekurrente Aminosäure des Typs AAI enthält.

11. Verfahren nach Anspruch 9, **dadurch gekennzeichnet, daß** mindestens ein Wirkstoff in der Flüssigkeit gelöst wird, vorzugsweise vor dem Einführen der Polyaminsäuren in das Medium. so daß nach diesem Einführen eine Wirkstoffhaltige Partikel-Kolloidlösung erzielt wird.

12. Verfahren nach Anspruch 9, **dadurch gekennzeichnet, daß** es mindestens einen zusätzlichen Verfahrensschritt zur Aggregation der Partikel mit Hilfe von mindestens einem Aggregationsmittel, vorzugsweise durch ein Salz und/oder eine Säure und/oder eine Base und/oder ein eventuell ionisches Polymer, enthält.

13. Verfahren nach Anspruch 12, **dadurch gekennzeichnet, daß** Polymerkonzentrationen in der Lösung, die in Gewichtsanteilen pro Volumen ausgedrückt mindestens 10⁻² betragen und vorzugsweise zwischen 0,05 und 30 und noch besser zwischen 0,05 und 5 liegen, gewählt werden.

14. Partikel nach Anspruch 1 und/oder mittels des Verfahrens nach Anspruch 9 hergestellte Partikel, **dadurch gekennzeichnet, daß** der Wirkstoff ein medikamentöser Stoff ist und vorzugsweise unter:
• den Proteinen und/oder den Peptiden, dabei vorzugsweise den Hämoglobinen, den Cytochromen, den Albuminen, den Interferonen, den Antigenen, den Antikörpern, dem Calatonin, dem Erythropoietin, dem Insulin, den Wachstumshormonen, dem IX-Faktor, dem Interleukin, oder deren Gemischen,
• den Polysacchariden, dabei insbesondere Heparin,
• den Nukleinsäuren und bevorzugt den ARN- und/oder ADN-Oligonukleotiden,
• und deren Gemischen
gewählt wird.

15. Partikel nach Anspruch 1 und/oder mittels des Verfahrens nach Anspruch 9 hergestellte Partikel, **dadurch gekennzeichnet, daß** der Wirkstoff aus mindestens einem Vaccin besteht.

16. Partikel nach Anspruch 1 und/oder mittels des Verfahrens nach Anspruch 9 hergestellte Partikel, **dadurch gekennzeichnet, daß** der Wirkstoff aus mindestens einem Pflanzenschutzoder Kosmetikprodukt besteht.

17. Spezialprodukt auf dem Gebiet der Pharmazie, der Ernährung, des Pflanzenschutzes oder der Kosmetik, **dadurch gekennzeichnet, daß** es Partikel nach Anspruch 1 und/oder mittels des Verfahrens nach Anspruch 9 hergestellte Partikel enthält.

## Claims

1. Nanoparticles (DNP) for the delivery of active principle(s), of the type based on polyaminoacid(s),
characterized:
- in that they have an average size comprised between 0.01 and 0.5 µm, preferably between 0.03 and 0.4 µm;
- in that these DNP are obtained by contacting polyaminoacids (**PAA**) with an aqueous solution;
- in that these **PAA** are linear with α-peptide linkages and comprise at least two types of recurring amino acids, AAN and AAI:
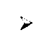 the type AAN corresponding to a hydrophobic neutral amino acid,
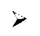 and the type AAI corresponding to an amino acid having an ionizable side chain, at least a portion of the type AAI recurring amino acids being in ionized form ;
the recurring amino acids of each type, AAN and AAI, being identical to or different from one another;
- in that the weight average molar mass M_{w} of the PAA is not less than 4,000 D, and preferably not less than 5,000 D ;
- and in that these **PAA** are non-hydrosoluble at acid pH and at pH comprised between 3 and 13 .

2. Particles according to claim 1, characterized in that their constituent polyaminoacids are block and/or statistical **PAA**, and in that :
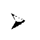 for the block **PAA**:
• the ratio AAN/AAN+AAI mole ratio is≥6%, preferably ≥ 15 % ,
• and M_{w} .≥.5,500 D, preferably 6,500 D≤ M_{w} ≤ 200,000 D and more preferably , 8,000 D ≤ M_{w}≤ 200,000 D ;
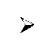 and for the statistical **PAA**:
• the AAN/AAN+AAI mole ratio is ≥ 20 % ,preferably ≥ 25 % ;
• and M_{w}≥ 10,000 D , preferably 20,000 D≤ M_{w} ≤ 500,000 D and more preferably , 200,000 D ≤ M_{w} ≤ 150,000 D .

3. Particles according to claim 1 or 2 , characterized in that
- the AAN (or the AANs) is (are) selected in the following list : Leu, lle, Val, Ala, Pro, Phe and mixtures thereof,
- and the AAI (or the AAls) is (are) Glu and/or Asp.

4. Particles according claim 1 or 2 , characterized in that the PAA comprises an recurrent aminoacid AAN and an recurrent aminoacid AAl.

5. Particles according to claim 1, having an average PAA concentration varying from 0.01 to 25% dry weight, preferably from 0.05 to 10% dry weight.

6. Particles according to claim 1, characterized in that they are delivery MicroParticles (**MPV**) having an average size > 0.5 µm , preferably ≤ 20 µm.

7. Particles according to claim 6, characterized in that they are obtained from particles according to claim 5 and that they comprise , preferably , at least one aggregating agent.

8. Particles according to claim 1 , characterized in that they comprise at least one active principle.

9. Method for preparing particles , based on polyamino acid(s), and which are capable of being used as delivery vehicles for active principles, characterized :
- in that polyaminoacids (**PAA**) are implemented :
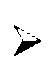 said PAA being linear with α-peptid linkages ,
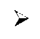 said PAA being comprising at least two types of recurring aminoacids , AAN and AAI:
• the type AAN corresponding to a hydrophobic neutral aminoacid ,
• and the type AAI corresponding to an aminoacid , having an ionisable side chain ,
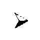 the AAN/AAI+AAN mole ratio being ≥ 3 % , preferably ≥ 5 %,
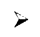 the weight average molar mass M_{w} of the polyaminoacid(s) being ≥ to 4,000 D, preferably > 5,000 D ,
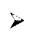 said "PAA being non-hydrosoluble at pH comprised between 3 and 13,
- in that a dispersion of these polyaminoacids is produced in an liquid , preferably in an aqueous solution ,
- and in that a colloidal solution of particles is produced.

10. Method according to claim 9 characterized in that a PAA comprising a recurrent aminoacid AAN and a recurrent aminoacid AAI.

11. Method according to claim 9, characterized in that at least one active principle is dissolved in the liquid , preferably before the introduction of the polyaminoacids , so that a colloidal solution of particles loaded with active principle(s) is obtained , after this introduction.

12. Method according to claim 9, characterized in that it comprises at least one additional step of aggregating the particles, by means of at least one aggregating agent, preferably constituted by a salt and /or an acid and / or a base and/or a polymer, eventually a ionic polymer.

13. Method according to claim 12, characterized in that the polymer concentrations in the solution are choosen not less than 10⁻² % weight/volume , preferably comprised between 0.05 and 30 % weight/volume and , more preferably comprised between 0.05 and 5 % weight/volume .

14. Particles according to claim 1 and/or obtained by the process according claim 9, characterized in that the active principle is medicinal and is preferably selected from :
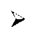 proteins and/or peptides among which the most preferably selected are the haemoglobins, the cytochromes, the albumins,the interferons, the antigens, the antibodies, the calcitonin, the erythropoietin, the insulin,the growth hormones,the factor IX and the interleukin or mixtures thereof;
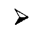 polysaccharides, the heparin being more particularly selected ;
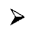 nucleic acids, and , preferably , the oligonucleotides of RNA and/or DNA;
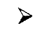 and mixtures thereof.

15. Particles according to claim 1 and/or obtained by the process according claim 9, characterized in that the active principle consists of at least one vaccine.

16. Particles according to claim 1 and/or obtained by the process according claim 9, characterized in that the active principle is composed of at least one plant-protection or cosmetic product.

17. Pharmaceutical, nutritional , plant-protection or cosmetic speciality characterized in that it contains particles according to claim 1 and/or obtained by the method according to claim 9.
